# EUROPÄISCHE PATENTSCHRIFT

(11) **EP 2 007 700 B1**
(45) Veröffentlichungstag und Bekanntmachung des Hinweises auf die Patenterteilung: **18.11.2009**
(21) Anmeldenummer: 07727775.4
(22) Anmeldetag: 04.04.2007
(51) Int. Cl.: C07C 2/78

(54) **VERFAHREN ZUR HERSTELLUNG VON ACETYLEN DURCH PARTIELLE OXIDATION VON KOHLENWASSERSTOFFEN**
PROCESS FOR PREPARING ACETYLENE BY PARTIAL OXIDATION OF HYDROCARBONS
PROCÉDÉ DE PRODUCTION D'ACÉTYLÈNE PAR OXYDATION PARTIELLE D'HYDROCARBURES

(30) Priorität: 11.04.2006 US 402326
(43) Veröffentlichungstag der Anmeldung: 31.12.2008
(73) Patentinhaber: BASF SE, 67056 Ludwigshafen (DE)
(72) Erfinder: EHRHARDT, Kai, Rainer, 67346 Speyer (DE); POCHE, Raymond, Prairieville, Louisiana 70769 (US); SCULLIN, William R., Baton Rouge, Louisiana 70816 (US); HAYES, Michael L., Gonzales, Louisiana 70737 (US)
(74) Vertreter: Isenbruck, Günter
(86) Internationale Anmeldenummer: PCT/EP2007/053304
(87) Internationale Veröffentlichungsnummer: WO 2007/118797

(56) Entgegenhaltungen:
- GB-A- 673 493
- US-A- 5 824 834

## Beschreibung

Die vorliegende Erfindung betrifft ein Verfahren zur Herstellung von Acetylen aus Kohlenwasserstoffen durch partielle Oxidation, Spaltung im Lichtbogen, Spaltung im Plasma oder Pyrolyse von Kohlenwasserstoffen oder Kohle.

Acetylen wird industriell unter anderem nach dem von BASF entwickelten Verfahren hergestellt, das auf partieller Oxidation von .Kohlenwasserstoffen mit Sauerstoff beruht. Es ist ausführlich in "Ullmann's Encyclopedia of Industrial Chemistry, Sixth Edition, 2000, Electronic Release, Chapter 4.2.1" beschrieben.

Die beiden Einsatzstoffströme - der den Kohlenwasserstoff enthaltende Stoffstrom (generell üblicherweise gesättigte, leicht verdampfbare Kohlenwasserstoffe, Alkane in der Kettenlänge meist bis C10, bevorzugt Erdgas) einerseits sowie der den Sauerstoff enthaltende Stoffstrom andererseits - werden zuerst auf ca. 200°C bis 650°C vorgewärmt. Die Vorwärmtemperatur ist abhängig von dem eingesetzten Kohlenwasserstoff. Üblicherweise erfolgt eine Aufheizung bis in die Größenordnung der Zündtemperatur des Kohlenwasserstoffs. Bei der Verwendung von Erdgas liegt die Vorwärmtemperatur üblicherweise in einem Bereich von etwa 500°C bis 650°C. Anschließend werden die beiden Stoffströme gemischt und erst danach im Feuerraum in einer Flamme umgesetzt, die durch den so genannten Brennerblock stabilisiert wird.

Vorwärmung und Vormischung in Verbindung mit einer nachfolgenden Flammenreaktion bilden die Grundidee des BASF-Verfahrens zur partiellen Oxidation von Kohlenwasserstoffen mit Sauerstoff zur Herstellung von Acetylen. Durch diese Verfahrensführung wird die Reaktionsgeschwindigkeit der Oxidation der Kohlenwasserstoffe von der Mischgeschwindigkeit der Stoffströme unabhängig. Auf diese Weise kann die Verweilzeit in der Reaktionszone auf Werte reduziert werden, die wesentlich kleiner sind als die Halbwertzeit des thermodynamisch instabilen Acetylens. Entsprechend können durch das Verfahren die Acetylenausbeuten wesentlich gesteigert werden.

Die Vormischung muss schnell und unter Vermeidung von Rückströmung erfolgen, weil sich das Gemisch aufgrund der hohen Vorerwärmung sonst selbst entzündet. In einem solchen Fall brennt die Flamme nicht im Feuerraum, sondern in der Vormischzone und muss gelöscht werden, wozu Stickstoff zugegeben und die Sauerstoffzufuhr zum Reaktor unterbrochen wird. Das Gas, das bis zur erneuten Zündung der Flamme und der Einhaltung der Spezifikationen nach der Zündung durch den Reaktor strömt, wird abgefackelt.

Die Flammenreaktion bei Temperaturen über etwa 1500°C wird durch Eindüsen von Wasser oder Öl nach wenigen Millisekunden gequencht, d.h. die sehr schnelle Abkühlung auf zum Beispiel 90°C beziehungsweise 220°C bricht die Radikal-Kettenreaktion in der Flamme ab. Hierdurch wird der Abbau des thermodynamisch instabilen Zwischenprodukts Acetylen verhindert. Nach sehr langen Verweilzeiten (größer ca. 1 Sekunde) könnte praktisch kein Acetylen mehr gewonnen werden. Reaktionsprodukt ist das so genannte Spaltgas, das ein Gemisch aus Acetylen, Roh-Synthesegas (hauptsächlich H₂ und CO), Wasserdampf und Nebenprodukten, insbesondere Ruß, sowie höhere Kohlenwasserstoffe, ist.

Eine Verbesserung des Verfahrens mit Wasserquench ist in US 5,824,834 beschrieben, worin ein Verfahren beschrieben ist, das mit geschlossenem Wasserquench-Kreislauf arbeitet. Dadurch wird der Kontakt des mit Schadstoffen belasteten Prozesswassers mit der Atmosphäre verhindert.

Das erzeugte Spaltgas wird nachfolgend komprimiert und dann auf bekannte Weise aufgetrennt. Grundsätzlich gibt es für den Druck am Eintritt in die Kompression einen zulässigen Betriebsbereich, der z.B. durch die Auslegung der Apparate oder das Sicherheitskonzept festgelegt ist. Verlässt der Saugdruck den zulässigen Bereich, werden Schaltungen ausgelöst. So wird z.B. beim BASF-Verfahren aus sicherheitstechnischen Gründen der Saugdruck in einem Bereich geregelt, der stets größer als der Umgebungsdruck ist, und die Kompressoren werden bei zu tiefem Druck abgeschaltet. Analog werden bei zu hohem Saugdruck ein oder mehrere Reaktoren abgeschaltet.

Für den Ausgleich kleiner Schwankungen im Massenstrom des Reaktionsgasgemisches kann ein konventioneller Regler, beispielsweise ein PID-Regler mit träger Regelcharakteristik eingesetzt werden, um den Saugdruck konstant zu halten. Die träge Regelcharakteristik ist bevorzugt, um bei normalem Betrieb Regelschwingungen, die sich negativ auf die nachfolgenden Prozessstufen auswirken, zu vermeiden.

Durch den Ausfall eines Reaktors, beispielsweise bedingt durch Vorzündung oder Ausfall eines Kompressors, kommt es zu sprunghaften massiven Veränderungen des Massenstromes des Reaktionsgasgemisches. Diese können durch den konvention.ellen Regler mit träger Regelungscharakteristik nicht ausgeregelt werden, so dass der Saugdruck den zulässigen Bereich verlässt, was wiederum zur Abschaltung von weiteren Anlagenteilen und im schlimmsten Fall sogar zum kompletten Stillstand der Anlage führt.

Das oben geschilderte Problem tritt insbesondere bei modernen Anlagen zur Herstellung von Acetylen auf, bei denen das Volumen der Apparate, die Laständerungen puffen könnten, deutlich reduziert ist, weil insbesondere Elektrofilter zum Abscheiden von Ruß nicht mehr vorgesehen sind, indem die Aufgabe, wie in US 60/775,158 beschrieben, durch den Kompressor übernommen und weil die Investition für einen Gasometer vermieden werden soll.

Es war somit Aufgabe der Erfindung, den kontinuierlichen Betrieb einer Anlage zur Herstellung von Acetylen auch bei hohen zu verarbeitenden Massenströmen zu gewährleisten. Insbesondere sollte der kontinuierliche Betrieb auch für Anlagen mit niedrigerem Puffervolumen, insbesondere wegen Wegfalls von Elektrofiltern zur Rußabscheidung und eines Gasometers für das zu verdichtende Gas gewährleistet werden.

Die Lösung besteht in einem Verfahren zum kontinuierlichen Betrieb einer Anlage zur Herstellung von Acetylen aus Kohlenwasserstoffen durch partielle Oxidation, Spaltung im Lichtbogen oder Pyrolye von Kohlenwasserstoffen unter Erhalt eines Reaktionsgasgemisches, das über einen Kompressor geführt wird und wobei der Druck des Reaktionsgasgemisches auf der Saugseite des Kompressors mittels eines konventionellen Reglers in einem vorgegebenen Bereich geregelt wird, das dadurch gekennzeichnet ist, dass zusätzlich ein übergeordneter modellgestützter prädiktiver Regler eingesetzt wird, der auf sprunghafte Veränderungen des Massenstromes des Reaktionsgasgemisches, reagiert.

Die Erfindung ist nicht eingeschränkt bezüglich der konkreten Durchführung des Verfahrens: Wesentlich ist, dass ein Reaktionsgemisch erhalten wird, das über mindestens einen Kompressor geführt wird.

Beispielsweise kann es sich hierbei um ein Verfahren handeln, wie es in US 60/775,158 beschrieben ist, bei dem auf Nass-Elektrofilter, die häufige Ursache von Betriebsunterbrechungen sein können, verzichtet wird.

Erfindungsgemäß wird für die Regelung des Druckes des Reaktionsgasgemisches auf der Saugseite des Kompressors zusätzlich zu dem konventionellen Regler mit träger Regelungscharakteristik ein übergeordneter modellgestützter prädiktiver Regler eingesetzt, der auf sprunghafte Veränderungen des Massenstromes des Reaktionsgasgemisches reagiert. Sprunghafte Veränderungen des Massenstromes des Reaktionsgasgemisches können dabei quasi momentane Veränderungen des Massenstromes des Reaktionsgasgemisches, um mehr als 5 %, bevorzugt mehr als 10 % oder auch um mehr als 50 % desselben, sein.

Die Druckregelung des Reaktionsgasgemisches auf der Saugseite des Kompressors kann bevorzugt durch die Rückführung von komprimierten Gas auf die Saugseite über ein Regelorgan, insbesondere ein oder mehrere Regelventile, Schieber oder Klappen erfolgen, dass durch den konventionellen Regler gesteuert wird.

Der Druck des Reaktionsgemisches auf der Saugseite der Kompression kann bei entsprechender technischer Ausrüstung zusätzlich durch die Drehzahl des Kompressors geregelt werden, was den im Mittel zurückgeführten Massenstrom des Reaktionsgasgemisches reduziert.

Bei einer Betriebsstörung, insbesondere dem Ausfall eines Reaktors, die zu einer quasi momentanen Verminderung des Massenstromes des Reaktionsgasgemisches führt, wird die Regelung des Drucks des Reaktionsgasgemisches auf der Saugseite des Kompressors durch den übergeordneten modellgestützten prädiktiven Regler gesteuert, der den entfallenden Massenstrom des Reaktionsgasgemisches berechnet und über die Kennlinie desselben ein Regelorgan ansteuert, so dass Reaktionsgasgemisch von der Druckseite auf die Saugseite des Kompressors zurückgeführt wird.

Modellgestützte prädiktive Regler sind bekannt, und basieren auf der Vorhersage des zukünftigen Verlaufs der Regelgröße und der Regeldifferenz über einen vorgegebenen zeitlichen Prädiktionshorizont auf der Basis der bis zum aktuellen Zeitpunkt gemessenen und gespeicherten historischen Werte.

Bevorzugt berechnet der übergeordnete modellgestützte prädiktive Regler fortlaufend den produzierten Massenstrom des Reaktionsgasgemisches, insbesondere als Vielfaches des eingesetzten Massenstromes an Kohlenwasserstoffen im Verfahren der Herstellung von Acetylen durch partielle Oxidation.

Der übergeordnete modellgestützte prädiktive Regler ermittelt bevorzugt den verminderten Massenstrom an Reaktionsgasgemisch, dass die vermehrte Rückführung von komprimiertem Reaktionsgasgemisch auf die Saugseite des Kompressors entspricht, als Differenz des errechneten Massenstromes vor und nach Beginn der Störung.

Dabei stellt der übergeordnete modellgestützte prädiktive Regler insbesondere die Stellung des Regelorgans über den berechneten, verminderten Massenstrom des Reaktionsgasgemisches, die aktuelle Stellung des Regelorgans und die Kennlinie des Regelorgans ein, misst die Stellgröße und modifiziert über diese Funktion des übergeordneten modellgestützten prädiktiven Reglers das Stellsignal, des Regelorgans derart, dass der Störeinfluss kompensiert wird.

Darüber hinaus kann nach dem Eingriff des übergeordneten modellgestützten prädiktiven Reglers der konventionelle Regler und/oder die Drehzahlregelung des Kompressors die gegebenenfalls verbleibende Regeldifferenz, die aus der endlichen Genauigkeit der Berechnungen resultieren kann, korrigieren.

Bei Ausfall eines Kompressors in einer Anlage umfassend zwei oder mehrere parallel geschaltete Kompressoren kann der übergeordnete modellgestützte prädiktive Regler den reduzierten Massenstrom an komprimiertem Reaktionsgasgemisch berechnen und einen entsprechenden Massenstrom des Reaktionsgasgemisches von der Saugseite des Kompressors aus der Anlage ableiten, insbesondere zu einer Fackel oder zu einem Kraftwerk.

Insbesondere wird der von der Saugseite des Kompressors aus der Anlage abgeleitete Massenstrom des Reaktionsgasgemisches über ein Regelorgan, insbesondere ein Ventil, einen Schieber oder eine Klappe, eingestellt und die erforderliche Stellung des Regelorgans bei Auftreten einer Störung und durch den übergeordneten modellgestützten prädiktiven Regler über die berechnete Differenz, die aktuelle Stellung des Ventils und die Kennlinie eingestellt.

Der im vorliegenden Verfahren eingesetzte übergeordnete modellgestützte prädiktive Regler ist bevorzugt ein Feed-Forward-Regler.

Das erfindungsgemäße Verfahren hat den Vorteil, dass es einen kontinuierlichen Betrieb von Anlagen zur Herstellung von Acetylen in effektiver und verfahrenstechnisch einfacher Weise, insbesondere auch für Anlagen mit großer Kapazität, jedoch begrenztem Puffervolumen, insbesondere infolge Wegfalls von Elektrofiltern zur Russabschaltung sowie des Gasometers, gewährleistet. Indem ein Stillstand der Anlage vermieden wird, wird auch eine Produktionsunterbrechung in einer Vielzahl von zusätzlichen betroffenen, vor- und nachgeschalteten Anlagen vermieden. Der Apparateverschleiß durch An- und Abfahrvorgänge, insbesondere der Kompressoren, wird reduziert.

Durch den Eingriff des übergeordneten modellgestützten prädiktiven Reglers werden Störungen sicher abgefangen und so die Ausweitung der anfänglichen Störung verhindert. Hierdurch ist es ferner möglich, den konventionellen PID-Regler träge einzustellen als ohne übergeordneten Regler, was den Normalbetrieb ruhiger macht.

Die Erfindung wird im Folgenden anhand einer Zeichnung näher erläutert.

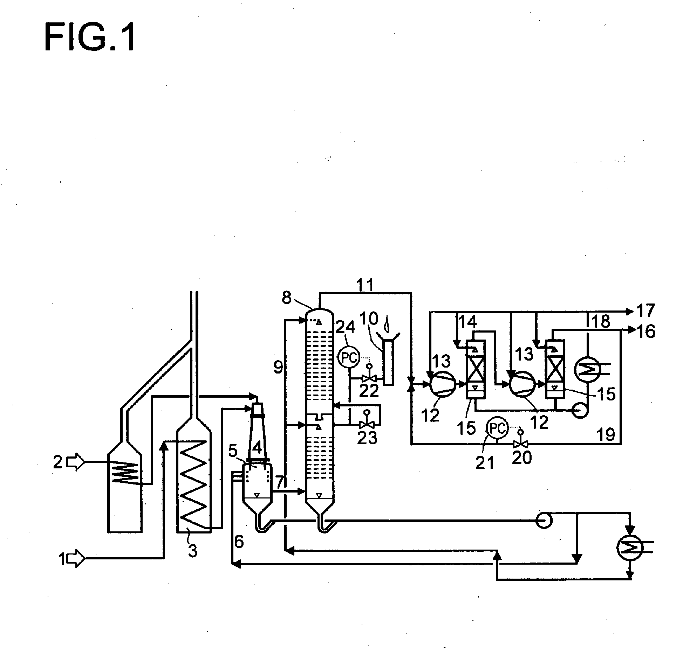

Die einzige Figur zeigt die schematische Darstellung einer bevorzugten Anlage zur Durchführung des erfindungsgemäßen Verfahrens.

Die Einsatzstoffe Erdgas 1 und Sauerstoff 2 werden in befeuerten Vorheizern 3 vorgewärmt, in der Mischzone 4 gemischt und im Feuerraum 5 in einer Flammenreaktion umgesetzt. Die Flamme wird unterhalb des Feuerraums durch Eindüsung von Prozesswasser gequencht. Das Acetylen enthaltende Reaktionsgasgemisch, das so genannte Spaltgas 7, tritt in etwa mit Kühlgrenztemperatur und mit Wasserdampf gesättigt in die Kühlkolonne 8 ein. Dort wird das Spaltgas mit Hilfe von gekühltem Prozesswasser gekühlt und dadurch ein Großteil des Wasserdampfes kondensiert. Die Fackel 10 wird für An- und Abfahrvorgänge sowie für Betriebsunterbrechungen benötigt, die beispielsweise durch Vorzündungen verursacht werden. Aus der Kühlkolonne 8 wird auf beispielhaft 40°C gekühltes Spaltgas 11 abgezogen und nachfolgend mittels eines zweistufigen Schraubenkompressors 12 mit Wassereinspritzung 13 von 1,1 auf 11 bar absolut komprimiert. Nach jeder Kompressionsstufe wird das Spaltgas mittels gekühltem Prozesswassers 14 in Kühlkolonnen 15 auf beispielhaft 40°C gekühlt. Das komprimierte Spaltgas 16 wird anschließend in seine Bestandteile aufgetrennt, wie beispielsweise in der eingangs zitierten Textstelle aus Ullmann's Encyclopedia of Industrial Chemistry, Sixth Edition, 2000, Electronic Release, Chapter 4.2.1, beschrieben.

Falls die Schraubenkompressoren mit konstanter Drehzahl laufen, ist die Differenz zwischen der verdichteten Spaltgasmenge (Strom 18) und der aus der Anlage abgezogenen Spaltgasmenge (Strom 16) auf die Saugseite der Kompressoren 12 zurückzuführen (Strom 19). Die rückgeführte Menge wird über den konventionellen Druckregler mit träger Regelcharakteristik 21 und das Regelventil 20 geregelt.

Falls der Schraubenkompressor 12 über eine Drehzahlregelung verfügt, kann zur Minimierung der Leistungsaufnahme die Fördermenge (Strom 18) über die Kompressordrehzahl angepasst werden. Drehzahlregelungen .sind jedoch langsam, weshalb auf eine zusätzliche Druckregelung nicht verzichtet werden kann.

Bei einer Betriebsstörung, bei der die Flamme aus dem Feuerraum 5 in die Mischzelle 4 zurückschlägt oder sich in der Mischzone das Kohlenwasserstoff-SauerstoffGemisch selbst entzündet, wird die Flamme durch Unterbrechung der O₂-Zufuhr (Strom 2) und N₂-Zugabe gelöscht und das Gas auf die Fackel 10 gestellt. Hierzu öffnet Fackelschieber 22 und schließt Anlagenschieber 23. Dies hat zur Folge, dass sich die über den Kompressoren 12 zugeführte Spaltgasmenge (Strom 11) praktisch sprunghafte vermindert, so dass die zurückgeführte Spaltgasmenge (Strom 19) entsprechend vergrößert werden muss. Ohne den schnellen Eingriff des Regelventils 20 wurde der Druck des Spaltgases auf der Saugseite des Kompressors (Strom 11) schnell abfallen, wobei die Geschwindigkeit der Druckreduktion von den Spaltgasvolumenströmen und dem Volumen der der Kompression vorgeschalteten Apparate und Rohrleitungen abhängt. Der schnelle Druckabfall kann eine Druckschaltung auslösen, die einen oder beide Kompressoren 12 abschaltet, wenn der Druck in einen Bereich unterhalb des vorgegebenen Bereichs abfällt.

Der Ausfall eines Kompressors, der,beispielsweise durch Schaltungen ausgelöst wird führt zu einer sprunghaften Verminderung des Spaltgasvolumenstromes (Strom 18), weshalb der saugseitige Spaltgasdruck des Stromes 11 ohne Gegenmaßnahmen schnell ansteigt. Übersteigt dieser Druck einen kritischen Wert, müssen Reaktoren abgeschaltet werden.

Es ist leicht ersichtlich, dass sich Störungen ausweiten können. Beispielsweise kann ein Brennerausfall zu einem unzulässigen Druckabfall führen, der die Abschaltung eines Kompressors verursacht. Daraufhin kann der Druck unzulässig ansteigen, was die Abspaltung weiterer Brenner verursacht. Letztlich ist auf diese Weise ein Totalausfall der Anlage, der zu einem beträchtlichen ökonomischen Schaden führt, durch eine vergleichsweise beschränkte anfängliche Störung möglich.

Für das Ausregeln dieser quasi momentanen anfänglichen Störung, die durch den konventionellen trägen Regler nicht zu bewerkstelligen ist und die wegen des verminderten Puffervolumens durch Wegfall eines Gasometers zwischen beispielsweise Kühlkolonne 8 und Kompressor 12 auch nicht aufgefangen werden kann, ist erfindungsgemäß beispielhaft eine übergeordnete Feed-Forward-Regelung vorgesehen. Diese ist zusätzlich zu dem konventionellen Druckregler 21 an derselben Stelle in der Anlage vorgesehen und daher in der Figur nicht zusätzlich dargestellt.

Die übergeordnete.Feed-Forward-Regelung berechnet fortlaufend die Spaltgasmenge (Strom 11) aus der zugeführten Erdgasmenge (Strom 1), wobei sie berücksichtigt, ob das Gas von einzelnen Reaktoren zur Kompression oder zur Fackel 10 geschickt wird. Bei der Berechnung wird die konkrete Zusammensetzung des eingesetzten Kohlenwasserstoffstromes berücksichtigt, das Verhältnis Sauerstoff zu Kohlenwasserstoffstrom sowie Temperatur und Druck des wasserdampfgesättigten Spaltgases.

Steht das Spaltgas von einem Reaktor, beispielsweise wegen einer Vorzündung, quasi momentan nicht mehr zur Kompression zur Verfügung, berechnet der übergeordnete Feed-Forward-Regler die Differenz wie oben dargelegt. Entsprechend der momentanen Stellung des Regelventils 20, der Kennlinie desselben und der berechneten Differenz der Spaltgasmengen vor und nach der Störung wird die neue Ventilöffnung des Regelventils 20 berechnet und direkt eingestellt. Danach wird die Druckregelung wieder von dem konventionellen, trägen Druckregler übernommen.

Fällt ein Kompressor aus, so wird die überschüssige Spaltgasmenge analog berechnet und der Fackelschieber 22 ebenso gezielt geöffnet. Danach wird die Druckregelung erneut von dem konventionellen trägen Druckregler 24 übernommen. Die Berechnung der komprimierten Spaltgasmenge berücksichtigt neben dem Fördervolumen des Kompressors bei Nenndrehzahl die aktuelle Kompressordrehzahl, sofern diese variabel ist. Wenn sich Druck und Temperatur am Eintritt des Kompressors und der Fackel nennenswert unterscheiden, sollte auch dies bei der Berechnung berücksichtigt werden. Ferner kann es je nach Kompressorbauart erforderlich sein, alle weiteren Parameter zu berücksichtigen, die die Fördermenge nennenswert beeinflussen, also die Kennlinie nennenswert verschieben. Ein Beispiel für Turboverdichter könnte der Druck nach der Kompression sein.

## Patentansprüche

1. Verfahren zum kontinuierlichen Betrieb einer Anlage zur Herstellung von Acetylen aus Kohlenwasserstoffen durch partielle Oxidation, Spaltung im Lichtbogen, Spaltung im Plasma oder Pyrolyse von Kohlenwasserstoffen oder Kohle unter Erhalt eines Reaktionsgasgemisches, das über einen Kompressor oder mehrere geführt wird und wobei der Druck des Reaktionsgasgemisches auf der Saugseite der Kompression mittels eines Reglers in einem vorgegebenen Bereich geregelt wird, **dadurch gekennzeichnet, dass** zusätzlich ein übergeordneter modellgestützter prädiktiver Regler eingesetzt wird, der auf sprunghafte Veränderungen des Massenstromes des Reaktionsgasgemisches reagiert.

2. Verfahren nach Anspruch 1, **dadurch gekennzeichnet, dass** der übergeordnete modellgestützte prädiktive Regler auf Veränderungen des Massenstromes des Reaktionsgemisches um mehr als 5%, bevorzugt mehr als 10 %, insbesondere um mehr als 50 % desselben, reagiert.

3. Verfahren nach Anspruch 1 oder 2, **dadurch gekennzeichnet, dass** der Druck des Reaktionsgasgemisches auf der Saugseite des Kompressors durch die Rückführung von komprimiertem Gas auf die Saugseite über ein Regelorgan, insbesondere ein oder mehrere Regelventile, Schieber oder Klappen erfolgt, das durch den konventionellen Regler gesteuert wird.

4. Verfahren nach Anspruch 3, **dadurch gekennzeichnet, dass** der Druck des Reaktionsgemisches auf der Saugseite des Kompressors zusätzlich durch die Drehzahl des Kompressors geregelt wird, die den im Mittel zurückgeführten Massenstrom des Reaktionsgasgemisches reduziert.

5. Verfahren nach einem der Ansprüche 1 bis 4, **dadurch gekennzeichnet, dass** bei einer Betriebsstörung, die zu einer quasi momentanen Verminderung des Massenstromes des Reaktionsgasgemisches führt, die Regelung des Druckes des Reaktionsgasgemisches auf der Saugseite des Kompressors durch den übergeordneten modellgestützten prädiktiven Regler gesteuert wird, der den entfallenden Massenstrom des Reaktionsgasgemisches berechnet und über die Kennlinie desselben ein Regelorgan ansteuert, so das Reaktionsgasgemisch von der Druck- auf die Saugseite des Kompressors zurückgeführt wird.

6. Verfahren nach einem der Ansprüche 1 bis 5, **dadurch gekennzeichnet, dass** der übergeordnete modellgestützte prädiktive Regler fortlaufend den produzierten Massenstrom des Reaktionsgasgemisches berechnet.

7. Verfahren nach Anspruch 6, **dadurch gekennzeichnet, dass** der übergeordnete modellgestützte prädiktive Regler den produzierten Massenstrom des Reaktionsgasgemisches als Vielfaches des angesetzten Massenstromes an Kohlenwasserstoffen im Verfahren der Herstellung von Acetylen durch partielle Oxidation berechnet.

8. Verfahren nach Anspruch 6, **dadurch gekennzeichnet, dass** der übergeordnete modellgestützte prädiktive Regler den verminderten Massenstrom an Reaktionsgasgemisch, der der vermehrten Rückführung von komprimiertem Reaktionsgasgemisch auf die Saugseite des Kompressors entspricht, als Differenz des errechneten Massenstromes vor und nach Beginn der Störung ermittelt.

9. Verfahren nach Anspruch 8, **dadurch gekennzeichnet, dass** der übergeordnete modellgestützte prädiktive Regler die Stellung des Regelorgans über den berechneten, verminderten Massenstrom des Reaktionsgasgemisches, die aktuelle Stellung des Regelorgans und die Kennlinie des Regelorgans einstellt, die Stellgröße misst und über diese Funktion des übergeordneten modellgestützten prädiktiven Reglers das Stellsignal des Regelorgans so modifiziert, dass der Störeinfluss kompensiert wird.

10. Verfahren nach einem der Ansprüche 1 bis 9, **dadurch gekennzeichnet, dass** nach dem Eingriff des übergeordneten modellgestützten pradiktiven Reglers der konventionelle Regler und/oder die Drehzahlregelung des Kompressors die gegebenenfalls verbleibende Regeldifferenz, die aus der endlichen Genauigkeit der Berechnungen resultieren kann, korrigiert.

11. Verfahren nach einem der Ansprüche 1 bis 4, **dadurch gekennzeichnet, dass** die Anlage zwei oder mehrere parallel geschaltete Kompressoren umfasst und dass der übergeordnete modellgestützte prädiktive Regler den reduzierten Massenstrom an komprimiertem Reaktionsgasgemisch bei Ausfall eines Kompressors oder mehrerer Kompressoren berechnet und einen entsprechenden Massenstrom des Reaktionsgasgemisches von der Saugseite der Kompression aus der Anlage ableitet, insbesondere zu einer Fackel oder zu einem Kraftwerk.

12. Verfahren nach Anspruch 11, **dadurch gekennzeichnet, dass** der von der Saugseite des Kompressors aus der Anlage abgeleitete Massenstrom des Reaktionsgemisches über ein Regelorgan, insbesondere ein Ventil, einen Schieber oder eine Klappe eingestellt wird und die erforderliche Stellung des Regelorgans bei Auftreten einer Störung durch den übergeordneten modellgestützten prädiktiven Regler über die berechnete Differenz, die aktuelle Stellung des Ventils und die Kennlinie eingestellt wird.

13. Verfahren nach einem der Ansprüche 1 bis 12, **dadurch gekennzeichnet, dass** der übergeordnete modellgestützte prädiktive Regler ein Feed-Forward-Regler ist.

14. Verfahren nach Anspruch 11, **dadurch gekennzeichnet, dass** der übergeordnete modellgestützte prädiktive Regler den komprimierten Massenstrom des Reaktionsgasgemisches aus der Kennlinie des Kompressors und aller Parameter, die die Kennlinie beeinflussen, insbesondere der Kompressordrehzahl, berechnet.

15. Verfahren nach Anspruch 11 oder 14, **dadurch gekennzeichnet, dass** der übergeordnete modellgestützte prädiktive Regler den aus der Anlage abzuleitenden Massenstrom des Reaktionsgasgemisches gemäß Druck und Temperatur am Eintritt des Kompressors und der Fackel und aus dem komprimierten Massenstroms des Reaktionsgasgemisches berechnet.

## Claims

1. A process for continuously operating a plant for preparing acetylene from hydrocarbons by partial oxidation, cleavage in an arc, cleavage in plasma or pyrolysis of hydrocarbons or carbon to obtain a reaction gas mixture which is conducted through one or more compressors, the pressure of the reaction gas mixture on the suction side of the compression being controlled within a predefined range by means of a controller, which comprises additionally using a higher-level model-supported predictive controller which reacts to abrupt changes in the mass flow rate of the reaction gas mixture.

2. The process according to claim 1, wherein the higher-level model-supported predictive controller reacts to changes in the mass flow rate of the reaction gas mixture by more than 5%, preferably more than 10%, in particular by more than 50% thereof.

3. The process according to claim 1 or 2, wherein the pressure of the reaction gas mixture on the suction side of the compressor is controlled by the recycling of compressed gas to the suction side by means of a control unit, in particular one or more control valves, vanes or flaps, which is controlled by the conventional controller.

4. The process according to claim 3, wherein the pressure of the reaction mixture on the suction side of the compressor is additionally controlled by the speed of the compressor which reduces the mass flow rate of the reaction gas mixture returned on average.

5. The process according to any of claims 1 to 4, wherein, in the event of a disruption to operation which leads to a quasi-instantaneous decrease in the mass flow rate of the reaction gas mixture, the pressure of the reaction gas mixture on the suction side of the compressor is controlled by the higher-level model-supported predictive controller which calculates the decrease in the mass flow rate of the reaction gas mixture and uses the characteristic thereof to actuate a control unit, so that reaction gas mixture is returned from the pressure side to the suction side of the compressor.

6. The process according to any of claims 1 to 5, wherein the higher-level model-supported predictive controller continually calculates the produced mass flow rate of the reaction gas mixture.

7. The process according to claim 6, wherein the higher-level model-supported predictive controller calculates the produced mass flow rate of the reaction gas mixture as a multiple of the mass flow rate of hydrocarbons used in the process for preparing acetylene by partial oxidation.

8. The process according to claim 6, wherein the higher-level model-supported predictive controller determines the reduced mass flow rate of reaction gas mixture which corresponds to the increased recycling of compressed reaction gas mixture to the suction side of the compressor as the difference of the calculated mass flow rate before and after the start of the disruption.

9. The process according to claim 8, wherein the higher-level model-supported predictive controller adjusts the position of the control unit using the calculated, reduced mass flow rate of the reaction gas mixture, the current position of the control unit and the characteristic of the control unit, measures the parameter and uses this function of the higher-level model-supported predictive controller to modify the control signal of the control unit so as to compensate for the disruptive influence.

10. The process according to any of claims 1 to 9, wherein, after the intervention of the higher-level model-supported predictive controller, the conventional controller and/or the speed controller of the compressor correct any control difference remaining, which can result from the finite precision of the calculations.

11. The process according to any of claims 1 to 4, wherein the plant comprises two or more compressors connected in parallel and the higher-level model-supported predictive controller calculates the reduced mass flow rate of compressed reaction gas mixture in the event of failure of one compressor or a plurality of compressors and diverts a corresponding mass flow rate of the reaction gas mixture from the suction side of the compression out of the plant, in particular to a flare or to a power station.

12. The process according to claim 11, wherein the mass flow rate, diverted from the suction side of the compressor out of the plant, of the reaction mixture is adjusted by means of a control unit, in particular a valve, a vane or a flap, and the required position of the control unit in the event of occurrence of disruption is adjusted by the higher-level model-supported predictive controller using the calculated difference, the current position of the valve and the characteristic.

13. The process according to any of claims 1 to 12, wherein the higher-level model-supported predictive controller is a feed-forward controller.

14. The process according to claim 11, wherein the higher-level model-supported predictive controller calculates the compressed mass flow rate of the reaction gas mixture from the characteristic of the compressor and all parameters which influence the characteristic, in particular the compressor speed.

15. The process according to claim 11 or 14, wherein the higher-level model-supported predictive controller calculates the mass flow rate of the reaction gas mixture to be diverted out of the plant according to pressure and temperature at the inlet of the compressor and the flare and from the compressed mass flow rate of the reaction gas mixture.

## Revendications

1. Procédé de conduite en continu d'une installation de préparation d'acétylène par oxydation partielle d'hydrocarbures, dissociation d'hydrocarbures dans un arc lumineux, dissociation d'hydrocarbures dans un plasma ou pyrolyse d'hydrocarbures ou de charbon, pour obtenir un mélange de gaz de réaction qui est amené dans un ou plusieurs compresseurs, la pression du mélange de gaz de réaction sur le côté d'aspiration de la compression étant régulée dans une plage prédéterminée au moyen d'un régulateur,
**caractérisé en ce que**
le procédé utilise en supplément un régulateur prédictif d'ordre hiérarchique supérieur et soutenu par modèle qui réagit à des modifications brusques du débit massique du mélange de gaz de réaction.

2. Procédé selon la revendication 1, **caractérisé en ce que** le régulateur prédictif d'ordre hiérarchique supérieur et soutenu par modèle réagit à des modifications du débit volumique du mélange de réaction de plus de 5 %, de préférence de plus de 10 %, et en particulier de plus de 50 %.

3. Procédé selon les revendications 1 ou 2,
**caractérisé en ce que** la pression du mélange de gaz de réaction sur le côté d'aspiration du compresseur est régulée par le renvoi de gaz comprimé vers le côté d'aspiration par un organe de régulation, en particulier une ou plusieurs soupapes ou un ou plusieurs registres ou clapets de régulation, et est commandée par un régulateur classique.

4. Procédé selon la revendication 3, **caractérisé en ce que** la pression du mélange de réaction sur le côté d'aspiration du compresseur est de plus régulée par le régime de rotation du compresseur, qui réduit le débit volumique moyen du mélange gaz de réaction renvoyé.

5. Procédé selon l'une des revendications 1 à 4,
**caractérisé en ce qu'**en cas de perturbation du fonctionnement qui entraîne une diminution quasi-instantanée du débit volumique du mélange de gaz de réaction, la régulation de la pression du mélange de gaz de réaction sur le côté d'aspiration du compresseur est commandée par le régulateur prédictif d'ordre hiérarchique supérieur et soutenu par modèle qui calcule la chute du débit volumique du mélange de gaz de réaction et commande en fonction de la ligne caractéristique de ce débit un organe de régulation de telle sorte que le mélange de gaz de réaction soit renvoyé du côté refoulement au côté aspiration du compresseur.

6. Procédé selon l'une des revendications 1 à 5,
**caractérisé en ce que** le régulateur prédictif d'ordre hiérarchique supérieur et soutenu par modèle calcule en permanence le débit massique de production du mélange de gaz de réaction.

7. Procédé selon la revendication 6, **caractérisé en ce que** le régulateur prédictif d'ordre hiérarchique supérieur et soutenu par modèle calcule le débit massique de la production du mélange de gaz de réaction en tant que multiple du débit massique d'hydrocarbures appliqués dans le procédé de fabrication d'acétylène par oxydation partielle.

8. Procédé selon la revendication 6, **caractérisé en ce que** le régulateur prédictif d'ordre hiérarchique supérieur et soutenu par modèle détermine la diminution du débit massique de mélange de gaz de réaction qui correspond à l'augmentation du renvoi de mélange de gaz de réaction comprimé vers le côté d'aspiration du compresseur sous la forme de la différence entre les débits massiques calculés avant et après le début de la perturbation.

9. Procédé selon la revendication 8, **caractérisé en ce que** le régulateur prédictif d'ordre hiérarchique supérieur et soutenu par modèle règle la position de l'organe de régulation par l'intermédiaire de la diminution calculée du débit massique du mélange de gaz de réaction, la position effective de l'organe de régulation et la ligne caractéristique de l'organe de régulation, mesure la grandeur de réglage et modifie par l'intermédiaire de cette fonction du régulateur prédictif d'ordre hiérarchique supérieur et soutenu par modèle le signal de réglage de l'organe de régulation de manière à compenser l'influence perturbatrice.

10. Procédé selon l'une des revendications 1 à 9,
**caractérisé en ce qu'**après l'intervention du régulateur prédictif d'ordre hiérarchique supérieur et soutenu par modèle, le régulateur classique et/ou la régulation du régime de rotation du compresseur corrigent l'éventuelle différence de réglage résiduel qui peut résulter du fait que les calculs ont une précision finie.

11. Procédé selon l'une des revendications 1 à 4,
**caractérisé en ce que** l'installation comprend deux ou plusieurs compresseurs raccordés en parallèle et **en ce que** le régulateur prédictif d'ordre hiérarchique supérieur et soutenu par modèle calcule la diminution du débit massique de mélange de gaz de réaction comprimé en cas de défaillance d'un ou de plusieurs compresseurs et dérive dans l'installation, en particulier vers une torche ou une centrale électrique, un débit massique approprié de mélange de gaz de réaction du côté d'aspiration du compresseur.

12. Procédé selon la revendication 11, **caractérisé en ce que** le débit massique de mélange de régulation prélevé sur l'installation par le côté d'aspiration du compresseur est réglé au moyen d'un organe de réglage, en particulier une soupape, un registre ou un clapet, et **en ce que** lorsqu'une perturbation survient, la position nécessaire de l'organe de régulation est établie par le régulateur prédictif d'ordre hiérarchique supérieur et soutenu par modèle par l'intermédiaire de la différence calculée, de la position effective de la soupape et de la ligne caractéristique.

13. Procédé selon l'une des revendications 1 à 12, **caractérisé en ce que** le régulateur prédictif d'ordre hiérarchique supérieur et soutenu par modèle est un régulateur dit « feed-forward ».

14. Procédé selon la revendication 11, **caractérisé en ce que** le régulateur prédictif d'ordre hiérarchique supérieur et soutenu par modèle calcule le débit massique comprimé du mélange de gaz de réaction à partir de la ligne caractéristique du compresseur et de tous les paramètres qui agissent sur la ligne caractéristique, en particulier le régime de rotation du compresseur.

15. Procédé selon les revendications 11 ou 14, **caractérisé en ce que** le régulateur prédictif d'ordre hiérarchique supérieur et soutenu par modèle calcule le débit massique de mélange de réaction qui doit être prélevé sur l'installation en fonction de la pression et de la température à l'entrée du compresseur et de la torche et à partir du débit massique de mélange de gaz de réaction comprimé.
